# EUROPEAN PATENT SPECIFICATION

(11) **EP 1 852 398 B1**
(45) Date of publication and mention of the grant of the patent: **01.07.2009**
(21) Application number: 06425152.3
(22) Date of filing: 08.03.2006
(51) Int. Cl.: C02F 1/46, C02F 1/48, A61L 2/03, A23L 3/32

(54) **Method and apparatus for the treatment with electric current of polluted fluids**
Verfahren und Vorrichtungen zur Behandlung beschmutzter Flüssigkeiten mittels eines elektrischen Stroms
Procédé et dispositifs de traitement de fluides pollués par par un courant électrique

(43) Date of publication of application: 07.11.2007
(73) Proprietor: Antomag s.r.l., 20135 Milano (MI) (IT)
(72) Inventor: Boschi, Cristiano, 06010 Citerna (PG) (IT)
(74) Representative: Concone, Emanuele

(56) References cited:
- EP-A- 0 553 377
- EP-A1- 1 000 554
- US-A- 3 766 050
- US-A- 4 169 029
- US-A- 5 326 530

## Description

The present invention relates to a method and an apparatus for the treatment with electric current of polluted fluids, typically liquids containing biological pollutants (viruses, bacteria, fungi, etc.) in variable amounts.

Known methods for the treatment with electric current of polluted liquids are based on the principle of the electric arc that is obtained by applying to the electrodes currents with low voltage and high amperage (from a few hundreds to thousands of Ampere, typical, for example, of metal welding, cutting and melting systems) to generate a plasma zone between the electrodes, with temperatures in the range of thousands of degrees centigrade, through which the polluted liquid is passed. An example of such a method is disclosed in US-A-4.169.029. These processes are generally intended to achieve the carbonization of the biological elements and inevitably imply the separation of hydrogen, oxygen and carbon contained in the liquids and in the electrodes thus producing a combustible gas mixed with water vapour as a by-product.

Hydrogen and oxygen, in fact, are the elements that make up water and are generated by electrolysis, while carbon is contained, in variable amounts, in the molecules of the polluting elements and in the electrodes used to generate the electric arc.

It is clear that said apparatuses have the drawback of requiring a very high consumption of electric power, as well as the need for replacing with a very high frequency the consumable electrodes required for the generation of the electric arc, usually of carbon and tungsten. These drawbacks are intrinsic in the electric arc method in that it is necessary to reach very high temperatures in the electrodes and especially between the electrodes in order for the carbonization of the biological components to occur.

Moreover, these methods have the further drawback that, in order to have the whole flow pass through the central plasma zone between the electrodes, it is necessary to limit the flow or to enlarge the electrode size. In both cases, this results absolutely counterproductive from the point of view of power consumption, even assuming the recovery of the combustible gas by separating it from the water vapour.

The applicant has found, on the basis of experiments carried out on the electric arc and on the plasma zone produced thereby, that in these systems the elimination of the biological component does not result so much and only from the current passing through the biological component but rather from the very high temperature that bums the component. Though being unable to exclude that the effects of the electric and magnetic field generated by the voltage and current passing through the portion of fluid flowing between the electrodes could cause a partial transformation of the biological components contained in the liquid to be treated, in these systems there is detected a high increase in carbon content in the outflowing liquid.

This increase is mainly caused by the high temperature between the electrodes (more than 3550°C) that causes the melting of the electrodes and the partial carbonization of the biological components, since a fraction of the flow passes through the plasma zone.

The applicant, through the present invention, aims at achieving the decrease and/or complete removal of the biological components present in a liquid by using only the current required to damage the biological components without having to use, and therefore waste, the electric power required to generate great amounts of heat and/or to keep an electric arc constantly active.

It is known that the electric current flowing through a biological organism provokes various changes of chemical and physical nature, due to different phenomena such as the polarization and/or rupture of the cellular membranes caused by the heat dissipated by Joule effect or by the action of the electric field, the denaturation of the proteins by rupture of the bond of the carboxyl group in the amino acids sequence, electrolysis, necrosis, etc. It is commonly believed that these damages to the biological structures are proportional to the voltage and amperage values of the electric current passing therethrough.

Actually, the biological damage depends on several other variables and not only on voltage and amperage, among which:
- type of wave: e.g. sinusoidal, square, continuous, pulsating;
- frequency;
- impedance of the conductor;
- period of exposure;
- type of conductivity, i.e. electronic flow or electrochemical conductivity.

Also the type of biological organism present in the polluted liquid is a variable, in that each cell has a threshold (called "rheobasis") beyond which the polarization/depolarization of the cell occurs. As a consequence, applying an electric current to a liquid polluted by biological organisms can cause the rupture of the cellular membranes and of the elements inside the cell, even at molecular level.

Based on this principle, a method has been developed that provides to achieve the rupture of the cellular membranes of the biological components present in the polluted liquid by subjecting the biological components to a rapid series of passages through electric currents that are different from each other and suitably adjusted in the parameters of voltage, amperage, frequency, wave shape, phase, DC/AC.

The applicant has thus built an apparatus for the treatment of liquids containing biological pollutants intended to eliminate, or at least decrease/modify, in a selective or global manner the pollutants while preferably obtaining as a by-product a limited amount of "clean" combustible gas, i.e. made up of hydrogen and oxygen with only negligible traces of carbon and without the presence of water vapour.

Therefore the object of the present invention is to provide a method and an apparatus which overcome the drawbacks of known devices. This object is achieved by means of a method that provides applying consecutively at least two different voltages that are different in absolute value by at least 50% and are anyway not lower than 45 V by passing this liquid through at least two conductive sections at which no electric arc is generated, and a corresponding apparatus including at least two conductive sections at which said voltages are applied and through which the liquid to be treated is passed. Other advantageous features of the present method and apparatus are disclosed in the attached claims.

The main advantage of the method and of the apparatus according to the present invention is that of performing the treatment of the polluted liquids by applying to the liquid flow suitable electric currents with very low amperage, without therefore generating an electric arc and without therefore any need for consumable electrodes and with a significantly low power consumption.

A second advantage of the present invention stems from the fact that the whole flow of liquid passing through the apparatus is subjected to the actions of the electric currents and therefore it is all treated in the same way and in a single passage.

A third significant advantage of the present invention results from its extreme flexibility, since the treatment can be specifically tailored to the type of pollutants by merely changing one or more parameters of the applied currents.

Still another advantage of this apparatus is its structural simplicity, that makes it cheap and reliable.

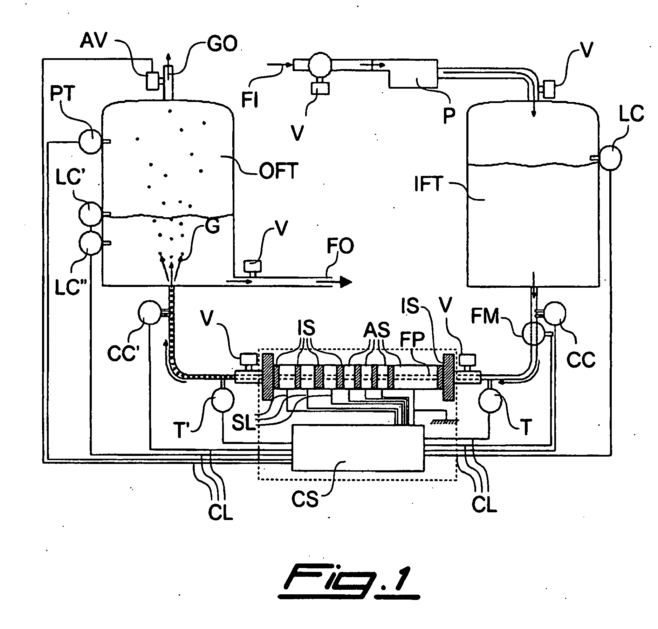

These and other advantages and characteristics of the method and of the apparatus according to the present invention will be clear to those skilled in the art from the following detailed description of an embodiment thereof, with reference to the only drawing, annexed as fig.1, that schematically illustrates a plant for the treatment of polluted fluids including said apparatus.

With reference to said figure, there is seen that an apparatus for the treatment of polluted fluids according to the present invention (indicated by the frame in broken lines) essentially consists of a plurality of electrically conductive rings or conductive sections AS spaced by suitable insulating sections IS, all having a central bore of the same diameter. The conductive sections AS, through supply lines SL, receive each a specific electric current generated by an electronic control system CS. The fluid to be treated passes through sections AS, IS in a fluid passage FP formed of the alternating sequence of insulating sections and conductive sections, so as to be subjected to the different conditions determined by the different types of current applied at the various conductive sections AS.

In the illustrated embodiment there are provided seven conductive sections AS of different lengths spaced by eight insulating sections IS also of different lengths (hatched areas). The number and length of the conductive sections AS changes according to the characteristics of the applied currents, and in the same way the insulating sections IS are sized according to the applied currents so as to prevent undesirable electric discharges between adjacent conductive sections.

As previously mentioned, the minimum number of conductive sections AS is two, in that it has been found that the apparatus begins to be effective already by simply applying to the liquid to consecutive currents with two different voltages where the second is at least 50% greater or smaller than the first, and both are at least 45 V.

In other words, naming X the voltage (either DC or AC) of the first conductive ring, the second conductive ring will receive a voltage equal to not more than 1/2 X or not less than 3/2 X (either DC or AC). This means that the fluid can be subjected to two direct currents or two alternating currents or firstly one and secondly the other, with a voltage that is decreased by half or increased by half, said parameters being selected according to the characteristics of the fluid to be treated. In order to increase the effectiveness of the treatment it is preferable that the currents supplied to two consecutive sections are in phase opposition, so as to cause a violent reversal of the polarization of the cells.

In a test carried out by the applicant on pighouse slurry there was achieved a decrease of over 99% in faecal coliforms and enterococci by using an apparatus with four conductive sections. In this case the current applied at the sections was mixed, with direct current generated by a rectifier at the first ring and alternating current at 50 Hz at the subsequent rings, and the voltage increasing in the direction of the liquid flow with values of 55 V, 110 V, 220 V and 440 V.

The treating apparatus is part of a treatment plant, whose structure depicted in fig.1 is merely exemplificative. The fluid passage FP is part of an hydraulic circuit in which the fluid enters through a fluid inlet FI and exits through a fluid outlet FO, passing through an inflowing fluid tank IFT and an outflowing fluid tank OFT. The circulation of the fluid is guaranteed by a pump P and controlled by a plurality of valves V.

The control system CS generates the electric currents to be supplied to the various conductive sections AS, through the supply lines SL, by setting the above-mentioned parameters, namely voltage, amperage, frequency, wave shape, phase and DC/AC. Furthermore, the control system CS is in communication with many other elements of the plant through communication lines CL that connect it to various types of sensors such as:
- thermometers T, T' to detect the temperature of the fluid entering and leaving the treatment apparatus;
- conductivity controllers CC, CC' to detect the conductivity of the fluid entering and leaving the treatment apparatus;
- a flow meter FM to detect the flow rate of the fluid entering and leaving the treatment apparatus;
- a level controller LC to detect the level of fluid in the inflowing fluid tank IFT;
- level controllers LC', LC" to detect the maximum and minimum level of fluid in the outflowing fluid tank OFT.

On the basis of the data collected through these sensors, the control system CS can adjust the characteristics of the currents applied at the conductive sections AS taking into account also the speed of the liquid flow (and therefore the period of exposure), the temperature and the conductivity.

In the preferred embodiment illustrated in fig.1 there is also provided the production of a mixture G of hydrogen and oxygen, as previously mentioned, that is released in the outflowing fluid tank OFT. To this purpose, said tank is also provided with a pressure transducer PT and with a gas outlet GO controlled by an adjustable valve AV, both the transducer PT and the valve AV being connected to the control system CS through respective lines CL.

It is clear that the above-described and illustrated embodiment of the apparatus according to the invention is just an example susceptible of various modifications. In particular, the number, shape and size of sections AS, IS can be freely changed according to the needs, as well as the materials used (e.g. stainless steel for sections AS, rubber for sections IS).

## Claims

1. Method for the treatment with electric current of fluids polluted by biological pollutants, including at least two steps in which two consecutive currents with two different voltages that differ in absolute value by at least 50% are applied to the fluid, **characterized in that** said at least two currents are applied to the fluid by passing it through at least two conductive sections at which no electric arc is generated, and **in that** said different voltages are not lower than 45 V.

2. Method according to claim 1, **characterized in that** the applied currents are DC and AC.

3. Method according to claim 1 or 2, **characterized in that** the currents applied in two consecutive steps of the treatment are in phase opposition.

4. Method according to one of the preceding claims, **characterized in that** the applied electric currents are individually controlled in one or more of their parameters, namely voltage, amperage, DC/AC, wave shape, phase, frequency, duration.

5. Method according to one of the preceding claims, **characterized in that** it includes a plurality of successive steps in which the voltage applied at each step is greater by at least 50% with respect to the voltage applied at the preceding step.

6. Apparatus for the treatment with electric current of fluids polluted by biological pollutants with the method according to one of the preceding claims, **characterized in that** it includes at least two conductive sections (AS) of electrically conductive material spaced by at least three insulating sections (IS), each of said conductive sections (AS) receiving through a supply line (SL) a specific electric current generated by an electronic control system (CS), a fluid passage (FP) through which the liquid to be treated is passed being formed by the alternating sequence of insulating sections (IS) and conductive sections (AS).

7. Plant for the treatment with electric current of fluids polluted by biological pollutants, **characterized in that** it includes an apparatus according to the preceding claim that is part of an hydraulic circuit in which the fluid enters through a fluid inlet (FI) and exits through a fluid outlet (FO) passing through an inflowing fluid tank (IFT) and an outflowing fluid tank (OFT), the circulation of the fluid in said plant being guaranteed by a pump (P) and controlled by a plurality of valves (V).

8. Plant according to the preceding claim, **characterized in that** it further includes a plurality of sensors such as thermometers (T, T') to detect the temperature of the fluid entering and leaving the treatment apparatus, conductivity controllers (CC, CC') to detect the conductivity of the fluid entering and leaving the treatment apparatus, a flow meter (FM) to detect the flow rate of the fluid entering and leaving the treatment apparatus and level controllers (LC, LC', LC") to detect the level of fluid in the fluid tanks (IFT, OFT), said sensors being operatively connected through communication lines (CL) to said control system (CS) of the treatment apparatus.

9. Plant according to claim 7 or 8, **characterized in that** the outflowing fluid tank (OFT) is also provided with a pressure transducer (PT) and with a gas outlet (GO) controlled by an adjustable valve (AV), both said transducer (PT) and said valve (AV) being operatively connected to the control system (CS) through respective communication lines (CL).

## Patentansprüche

1. Verfahren zur Behandlung von mit biologischen Kontaminanten verschmutzten Fluiden mittels elektrischen Stroms, das zumindest zwei Schritte einschließt, in welchen zwei aufeinander folgende Ströme mit zwei unterschiedlichen Spannungen, die sich im Absolutwert um zumindest 50 % unterscheiden, an das Fluid angelegt werden, **dadurch gekennzeichnet, dass** die zumindest zwei Ströme an das Fluid angelegt werden, indem dieses durch zumindest zwei leitfähige Abschnitte geführt wird, an welchen kein elektrischer Lichtbogen erzeugt wird, und dass die unterschiedlichen Spannungen nicht weniger als 45 V betragen.

2. Verfahren nach Anspruch 1, **dadurch gekennzeichnet, dass** die angelegten Ströme ein Gleichstrom und ein Wechselstrom sind.

3. Verfahren nach Anspruch 1 oder 2, **dadurch gekennzeichnet, dass** die in zwei aufeinander folgenden Schritten der Behandlung angelegten Ströme eine gegenteilige Phasenlage aufweisen.

4. Verfahren nach einem der vorhergehenden Ansprüche, **dadurch gekennzeichnet, dass** die angelegten elektrischen Ströme individuell in einem oder mehreren ihrer Parameter, nämlich Spannung, Stromstärke, Gleichstrom/Wechselstrom, Wellenform, Phase, Frequenz und Dauer gesteuert werden.

5. Verfahren nach einem der vorhergehenden Ansprüche, **dadurch gekennzeichnet, dass** es eine Vielzahl aufeinander folgender Schritte umfasst, in welchen die in jedem Schritt angelegte Spannung jeweils um zumindest 50% größer als die im vorhergehenden Schritt angelegte Spannung ist.

6. Vorrichtung zur Behandlung von mit biologischen Kontaminanten verschmutzten Fluiden mittels elektrischen Stroms mit dem Verfahren nach einem der vorhergehenden Ansprüche, **dadurch gekennzeichnet, dass** sie zumindest zwei leitfähige Abschnitte (AS) aus elektrisch leitfähigem Material umfasst, die durch zumindest drei Isolierabschnitte (IS) beabstandet sind, wobei jeder der leitfähigen Abschnitte (AS) über eine Versorgungsleitung (SL) einen durch ein elektronisches Steuersystem (CS) erzeugten spezifischen elektrischen Strom erhält, wobei ein Fluiddurchgang (FP), durch welchen die zu behandelnde Flüssigkeit geführt wird, aus einer abwechselnden Abfolge von Isolierabschnitten (IS) und leitfähigen Abschnitten (AS) gebildet wird.

7. Anlage zur Behandlung von mit biologischen Kontaminanten verschmutzten Fluiden mittels elektrischen Stroms, **dadurch gekennzeichnet, dass** sie eine Vorrichtung nach dem vorhergehenden Anspruch einschließt, welche einen Teil eines hydraulischen Kreises bildet, in welchem das Fluid durch einen Fluideinlass (FI) eintritt, einen Eingangsfluidtank (IFT) und einen Ausgangsfluidtank (OFT) durchläuft und durch einen Fluidauslass (FO) austritt, wobei die Umwälzung des Fluids in der Anlage durch eine Pumpe (P) gesichert und durch eine Vielzahl von Ventilen (V) gesteuert wird.

8. Anlage nach dem vorhergehenden Anspruch, **dadurch gekennzeichnet, dass** sie des Weiteren eine Vielzahl von Sensoren wie etwa Thermometer (T, T') zur Erfassung der Temperatur des in die Behandlungsvorrichtung eintretenden und diese verlassenden Fluids, Leitfähigkeitsregler (CC, CC') zur Erfassung der Leitfähigkeit des in die Behandlungsvorrichtung eintretenden und diese verlassenden Fluids, einen Durchflussmesser (FM) zur Erfassung der Strömungsrate des in die Behandlungsvorrichtung eintretenden und diese verlassenden Fluids sowie Niveauregler (LC, LC', LC") zur Erfassung des Fluidniveaus in den Fluidtanks (IFT, OFT) umfasst, wobei die Sensoren operativ über Kommunikationsleitungen (CL) mit dem Steuersystem (CS) der Behandlungsvorrichtung verbunden sind.

9. Anlage nach Anspruch 7 oder 8, **dadurch gekennzeichnet, dass** der Ausgangsfluiddtank (OFT) auch mit einem Druckmesswandler (PT) und mit einem durch ein einstellbares Ventil (AV) gesteuerten Gasauslass (GO) versehen ist, wobei sowohl der Messwandler (PT) als auch das Ventil (AV) operativ jeweils über Kommunikationsleitungen (CL) mit dem Steuersystem (CS) verbunden sind.

## Revendications

1. Procédé pour le traitement, à l'aide d'un courant électrique, de fluides pollués par des polluants biologiques, comprenant au moins deux étapes dans lesquelles deux courants consécutifs, ayant deux tensions différentes qui diffèrent en valeur absolue d'au moins 50 %, sont appliqués au fluide, **caractérisé en ce que** lesdits au moins deux courants sont appliqués au fluide en le faisant circuler à travers au moins deux tronçons conducteurs au niveau desquels aucun arc électrique n'est généré, et **en ce que** lesdites tensions différentes ne sont pas inférieures à 45 V.

2. Procédé selon la revendication 1, **caractérisé en ce que** les courants appliqués sont du courant continu (DC) et du courant alternatif (AC).

3. Procédé selon la revendication 1 ou 2, **caractérisé en ce que** les courants appliqués dans deux étapes consécutives du traitement ont des phases opposées.

4. Procédé selon l'une quelconque des revendications précédentes, **caractérisé en ce que** les courants électriques appliqués sont commandés individuellement dans une ou plusieurs de leurs paramètres, c'est-à-dire la tension, l'ampérage, DC/AC, la forme d'onde, la phase, la fréquence, et la durée.

5. Procédé selon l'une quelconque des revendications précédentes, **caractérisé en ce qu'**il comprend une pluralité d'étapes successives durant lesquelles la tension appliquée à chaque étape est supérieure d'au moins 50 % à la tension appliquée à l'étape précédente.

6. Appareil pour le traitement, à l'aide d'un courant électrique, de fluides pollués par des polluants biologiques, à l'aide du procédé selon l'une quelconque des revendications précédentes, **caractérisé en ce qu'**il comprend au moins deux tronçons conducteurs (AS) d'un matériau électriquement conducteur séparés par au moins trois tronçons isolants (IS), chacun desdits tronçons conducteurs (AS) recevant, à travers une ligne d'alimentation (SL), un courant électrique spécifique généré par un système de commande électronique (CS), un passage de fluide (FP) à travers lequel le liquide devant être traité circule, étant formé par la séquence alternée de tronçons isolants (IS) et de tronçons conducteurs (AS).

7. Installation pour le traitement, à l'aide d'un courant électrique, de fluides pollués par des polluants biologiques, **caractérisée en ce qu'**il comprend un appareil selon la revendication précédente qui est une partie d'un circuit hydraulique dans lequel le fluide entre à travers une entrée de fluide (FI) et sort à travers une sortie de fluide (FO) en passant à travers un réservoir de fluide d'écoulement entrant (IFT) et un réservoir de fluide d'écoulement sortant (OFT), la circulation du fluide dans ladite installation étant garantie par une pompe (P), et étant commandée par une pluralité de vannes (V).

8. Installation selon la revendication précédente, **caractérisée en ce qu'**elle comprend en outre une pluralité de capteurs comme des thermomètres (T, T') pour détecter la température du fluide entrant dans l'appareil de traitement, et le quittant, des contrôleurs de conductivité (CC, CC') pour détecter la conductivité du fluide entrant dans l'appareil de traitement, et le quittant, un débitmètre (FM) pour détecter le débit d'écoulement du fluide entrant dans l'appareil de traitement, et le quittant, et des contrôleurs de niveau (LC, LC', LC") pour détecter le niveau de fluide dans les réservoirs de fluide (IFT, OFT), lesdits capteurs étant reliés de manière opérationnelle, à travers des lignes de communication (CL), audit système de commande (CS) de l'appareil de traitement.

9. Installation selon la revendication 7 ou 8, **caractérisée en ce que** le réservoir de fluide d'écoulement sortant (OFT) est également muni d'un transducteur de pression (PT) et d'une sortie de gaz (GO) commandée par une vanne ajustable (AV), ledit transducteur (PT) et ladite vanne (AV) étant reliés de manière opérationnelle au système de commande (CS) à travers des lignes de communication respectives (CL).
